# EUROPÄISCHE PATENTANMELDUNG

(11) **EP 4 357 802 A1**
(43) Veröffentlichungstag der Anmeldung: **24.04.2024**
(21) Anmeldenummer: 22202927.4
(22) Anmeldetag: 21.10.2022
(51) Int. Cl.: G01R 33/28

(54) **VERFAHREN ZUM ENTWERTEN EINER LOKALSPULE FÜR EIN MRT-SYSTEM**

(71) Anmelder: Siemens Healthineers AG, 91301 Forchheim (DE)
(72) Erfinder: Dr. Biber, Stephan, 91056 Erlangen (DE)
(74) Vertreter: Siemens Healthineers Patent Attorneys

(57) **Zusammenfassung**

Die Erfindung betrifft ein Verfahren zum Entwerten einer Lokalspule (7) für ein MRT-System (1) umfassend die Schritte:
- Durchführen einer Messung mit der Lokalspule (7) an dem MRT-System (1),
- gezieltes Ändern einer mechanischen und/oder elektrischen Eigenschaft der Lokalspule (7) und/oder einer Information über die Lokalspule (7) im MRT-System (1) dahingehend, dass eine Anzahl der mit dieser Lokalspule (7) möglichen Messungen reduziert wird und in dem Fall, dass nur die durchgeführte Messung mit der Lokalspule (7) möglich war, eine weitere Messung mit dieser Lokalspule (7) zumindest an dem MRT-System (1) nicht mehr möglich ist.

Die Erfindung betrifft des Weiteren eine Lokalspule sowie ein MRT-System.

## Beschreibung

Die Erfindung betrifft ein Verfahren zum Entwerten einer Lokalspule für ein MRT-System (MRT: "Magnetresonanztomographie") sowie eine entsprechende Lokalspule und ein MRT-System. Insbesondere betrifft die Erfindung eine Anordnung und Verfahren zum Unbrauchbar-Machen von MRT-Lokalspulen nach einmaliger Verwendung. Die Erfindungsmeldung beschäftigt sich insbesondere mit dem Aufbau einer einmal benutzbaren Lokalspule und Verfahren zur gezielten Zerstörung von Lokalspulen nach ihrem Gebrauch.

An heutigen MRT-Systemen werden Aufnahmen oftmals unter Nutzung von Lokalspulen gemacht. Diese Lokalspulen befinden sich in, unter oder über dem aufzunehmenden Bereich und liefern die Messdaten, aus denen dann nach der Aufnahme die Bilder rekonstruiert werden. Die Lokalspule kann dabei aus einer einzigen Spule gebildet werden oder einer Anordnung von mehreren Spulen.

Insbesondere für bestimmte Aufnahmen, z.B. Dental oder in anderen Körperöffnungen gelten dabei besondere Anforderungen an die Hygiene. Gerade wenn hygienische Probleme auftreten können, ist die Verwendung von Einweg-Spulen (auch im Deutschen oft englisch als "Disposable Coils" bezeichnet) von großem Vorteil, da keine besonderen Anforderungen an eine Desinfektion gestellt werden müssen.

Bei Wegwerfspulen besteht ein großes Interesse daran, sicherzustellen, dass eine einmal verwendete Spule nach Benutzung nicht mehr verwendet wird. Damit wird sichergestellt, dass die Spule keine Krankheitserreger übertragen kann.

Es ist eine Aufgabe der vorliegenden Erfindung, ein Verfahren zum Entwerten einer Lokalspule für ein MRT-System anzugeben, welches die Nachteile des Standes der Technik überwindet und eine einfache Sicherstellung hygienischer Grundanforderungen erlaubt. Eine weitere Aufgabe ist die Bereitstellung einer entsprechenden Lokalspule bzw. eines MRT-Systems.

Diese Aufgabe wird durch ein Verfahren gemäß Patentanspruch 1, eine Lokalspule gemäß Patentanspruch 11 und ein MRT-System gemäß Patentanspruch 15 gelöst.

Das erfindungsgemäße Verfahren zum Entwerten einer Lokalspule für ein MRT-System umfasst die folgenden Schritte:
- Durchführen einer Messung mit der Lokalspule an dem MRT-System,
- optional: ermitteln einer Fertigstellung der Messung,
- gezieltes Ändern einer mechanischen und/oder elektrischen Eigenschaft der Lokalspule und/oder einer Information über die Lokalspule im MRT-System dahingehend, dass eine Anzahl der mit dieser Lokalspule möglichen Messungen reduziert wird und in dem Fall, dass nur (noch) die durchgeführte Messung mit der Lokalspule möglich war, eine weitere Messung mit dieser Lokalspule zumindest an dem MRT-System nicht mehr möglich ist.

Die Durchführung der Messung ist im Stand der Technik bekannt. Dazu wird die Lokalspule an einer festgelegten Position am Patienten bzw. dem zu untersuchenden Objekt platziert und der Patient bzw. das zu untersuchende Objekt in einem MRT-Scanner angeordnet. Danach wird die Messung durchgeführt.

Zu einem vorher festgelegten Zeitpunkt, insbesondere nach Fertigstellen der Messung aber nicht notwendigerweise, wird dann die Lokalspule "entwertet". Dazu wird gezielt eine mechanische und/oder elektrischen Eigenschaft der Lokalspule geändert, also z.B. ihre Spulenwicklung oder ein Bauteil der Lokalspule zerstört oder zumindest dessen Eigenschaften geändert. Dies kann auf mechanische Weise geschehen, z.B. durch eine einwirkende Kraft oder elektrisch z.B. durch einen Strom, der zum Durchschmelzen einer Sicherung oder des Spulenleiters führt. Es kann auch eine Information über die Lokalspule im MRT-System geändert werden, z.B. ein Zähler, der angibt, wie viele Messungen mit dieser Spule noch möglich sind, wobei das MRT-System in diesem Fall den/die Scanner und ggf. auch angeschlossene Server zur Organisation des MRT-Systems umfasst, welche entsprechende Daten zur Überprüfung der Lokalspule enthalten.

Die Änderung erfolgt dahingehend, dass eine Anzahl der mit dieser Lokalspule möglichen Messungen reduziert wird, also z.B. ein Zähler heruntergezählt wird. Bei einer Einweg-Spule wird dieser Zähler nur den Wert "1" aufweisen, also nur die durchgeführte Messung mit der Lokalspule möglich sein. In diesem Falle bedeutet die Reduktion der Anzahl der Messungen, dass keine weitere Messung mit dieser Lokalspule mehr möglich ist zumindest an dem betreffenden MRT-System. Dies kann z.B. mit besagter Zerstörung der Lokalspule erreicht werden. Die Änderung erfolgt dabei nach der Fertigstellung der Messung oder bereits nach dem Einstecken der Lokalspule, wobei in letzterem Falle die Messung noch durchgeführt werden sollte. Beispielsweise könnte nach dem Einstecken der Lokalspule in ihr Interface die Spule bereits durch herunterzählen eines Zählers entwertet werden. Sie wird jedoch zu diesem Zeitpunkt nicht zerstört, da ansonsten keine Messung mehr möglich wäre. Jedoch würde bei einem erneuten Einstecken das MRT-System erkennen, dass die Spule bereits entwertet ist.

Eine erfindungsgemäße Lokalspule für eine Messung mit einem MRT-System umfasst eine Änderungseinheit zum gezielten Ändern einer mechanischen und/oder elektrischen Eigenschaft der Lokalspule dahingehend, dass eine Anzahl der möglichen Messungen reduziert wird und in dem Fall, dass nur (noch) die durchgeführte Messung mit der Lokalspule möglich war, eine weitere Messung mit dieser Lokalspule an dem MRT-System nicht mehr möglich ist.

Dazu umfasst die Lokalspule bevorzugt eine Speichereinheit mit einem geschützten Speicher, dessen Inhalt durch ein Element der Lokalspule oder eines Interfaces geändert werden kann aber nicht durch einen Benutzer. Die Lokalspule kann bevorzugt eine RX-Spule oder eine TX-Spule oder eine RX/TX-Spule sein. Eine RX-Spule kann dazu ausgebildet sein, hochfrequente Signale, insbesondere Magnetresonanzsignale eines Patienten, zu empfangen. Eine TX-Spule kann dazu ausgebildet sein, hochfrequente Signale, insbesondere Signale zur Anregung eines Gewebes eines Patienten, auszusenden oder zu emittieren. Eine RX/TX-Spule kann insbesondere eine Spule darstellen, welche eine RX-Spule und eine TX-Spule umfasst.

Ein erfindungsgemäßes MRT-System umfasst eine Änderungseinheit zum gezielten Ändern einer mechanischen und/oder elektrischen Eigenschaft einer Lokalspule dahingehend, dass eine Anzahl der möglichen Messungen reduziert wird und in dem Fall, dass nur (noch) die durchgeführte Messung mit der Lokalspule möglich war, eine weitere Messung mit dieser Lokalspule an dem MRT-System nicht mehr möglich ist.

Die Lokalspule oder das MRT-System kann bevorzugt eine Einheit zur Ermittlung einer Fertigstellung der Messung, mit der Lokalspule an dem MRT-System umfassen, wobei die Lokalspule bzw. das MRT-System in diesem Fall bevorzugt dazu ausgelegt ist, dass nach Ermittlung einer Fertigstellung einer Messung automatisch das gezielte Ändern einer mechanischen und/oder elektrischen Eigenschaft der Lokalspule bewirkt wird.

Es ist bevorzugt, dass nach Fertigstellung einer Messung die Lokalspule durch gezielte Beeinflussung ihrer elektrischen bzw. mechanischen Strukturen in einen Zustand gebracht wird, der es nicht erlaubt, eine zweite Messung mit ihr durchzuführen. Dazu wird die Lokalspule bevorzugt in einen Zustand gebracht, in dem sie absichtlich durch einen Nutzer oder ein MRT-System unbrauchbar gemacht wurde, oder in einen Zustand gebracht wurde, in dem sie vom System nicht (mehr) erkannt wird oder in einen Zustand gebracht wurde, in dem sie vom System nicht (mehr) als gültig erkannt wird.

Ist die Spule z.B. über ein "Interface" als Zwischenkomponente mit dem MRT-System verbunden (dies ist meist ein Stecker am Patiententisch) verbunden. Dieses Interface übernimmt bevorzugt zusätzliche mechanische oder elektrische Funktionen zur gezielten Zerstörung der genutzten Lokalspule. Es gibt verschiedene Vorrichtungen und damit verbundene Verfahren, wie die Lokalspule entsprechend "geändert" werden kann.

Eine Änderung erfolgt bevorzugt, wenn eine Messung fertiggestellt ist. Bevorzugte Zeitpunkte zur Definition des "Fertigstellens der Messung" sind das Ausstecken der Spule ("entstecken") vom System oder vom Interface, das Ende der Messung, welches durch Kombination bestimmte typische Abläufe am System erkannt werden kann, z.B. PTAB Bewegung rein / Messung mit gewisser Dauer / PTAB Bewegung raus oder durch das System als explizites Messende festgelegt werden kann und bevorzugt definiert wird, bevor PTAB aus dem Bore herausgefahren werden kann. Das "Fertigstellen der Messung" kann auch als eine bestimmte Zeitspanne zwischen Erkennen der Spule / Start der Messung und dem Ablauf der Gültigkeit der Spule festgelegt werden. Es wird bevorzugt mindestens einer dieser "Endpunkte" einer Messung verwendet, um damit die Lebensdauer der Lokalspule zu begrenzen.

Es gibt unterschiedliche Maßnahmen für eine gezielte Zerstörung der Lokalspule. Bevorzugte hardware-basierte Maßnahmen sind das Durchtrennen des Hauptleiters der Lokalspule. Dies erfolgt bevorzugt über das Interface, kann aber auch ohne dieses erfolgen. Normalerweise wird die Lokalspule über einen Stecker mit dem Interface verbunden. Das Interface bildet eine mechanische und oder elektrische Schnittstelle zum MRT-System. Das bevorzugte Interface bildet nun einen mechanischen Eingriff in die Spule (kann z.B. als besagte Änderungseinheit angesehen werden) der beim Lösen, z.B. durch Ziehen oder Drücken eines Entriegelungsknopfes, des Interfaces dazu führt, dass der Hauptleiter der Lokalspule durchtrennt wird. Der Hauptleiter ist dazu bevorzugt auf einer kurzen Strecke freitragend innerhalb der Spule ausgeführt, so das der Leiter und seine dünne Tragestruktur leicht durchgebrochen werden kann, z.B. durch eine meißelartige Struktur (als Änderungseinheit), die beim Entriegeln oder Abziehen der Spule auf den Leiter drückt und diesen durchtrennt. Auch kann ein Abbrechen der Kontakte an der Spule selbst beim Trennen der Spule vom Interface hervorgerufen werden. Ebenso ist ein internes Kurzschließen des Hauptleiters der Spule oder von Teilen des Resonanzkreises bevorzugt. Dies kann auch durch eine Bewegung beim Entstecken hervorgerufen werden. Als elektrische Maßnahme kann die Lokalspule am Ende der Messung über das Interface mit einem hohen Strompuls versehen werden, welcher ein Bauteil in der Spule zerstört. Dies kann bevorzugt eine Schmelzsicherung sein. Solche Schmelzsicherungen werden zum Zwecke der Patientensicherheit in Spulen verwendet, um bei zu hohem HF-Strom über eine gewisse Dauer den Resonanzkreis der Spule zu unterbrechen und mögliche Verbrennungen des Patienten zu verhindern. Dieser Fall tritt aber höchst selten und nur bei Fehlbedienungen oder größeren Fehlern am System oder der Spule auf.

Es ist neben einer Zerstörung auch möglich, die Lokalspule zu verstimmen, also ihre Eigenfrequenz zu ändern. Dies erfolgt bevorzugt durch Öffnen einer Schmelzsicherung oder durch Bildung eines Kurzschlusses. Bevorzugt wird in diesem Rahmen ein parallel geschaltetes Bauteil, insbesondere eine Induktivität und/oder Kapazität, aktiviert oder deaktiviert und dadurch die Antenne der Lokalspule verstimmt. Ähnliches ist auch mit einer Diode möglich, die durch einen hohen Strom/Spannungspuls leitend gemacht wird und zu einem Kurzschluss der Spule führt.

Eine bevorzugte Änderung ist auch eine Änderung die zu einer Verhinderung der Spulenidentifikation durch das MRT-System führt. Die Lokalspule wird dadurch vom MRT-System nicht mehr erkannt. Falls diese Identifikation über eine elektrische Schnittstelle von Interface zu Lokalspule läuft, kann ähnlich wie beim Durchtrennen des Hauptleiters einer dieser Leiter unterbrochen werden und damit eine Identifikation verhindert werden. Bei Nutzung eines EEPROM kann diesbezüglich eine Trennung der Datenleitung oder auch der Leitung für den Übertragungstakt erfolgen. Es ist aber auch bevorzugt, den Spulencode durch mechanisches Unterbrechen oder Verbinden eines Kontaktes innerhalb der Spule beim Entstecken der Spule zu unterbrechen. Ebenso ist bevorzugt, dass durch das Unterbrechen oder Kurzschließen eines Leiters ein in der Lokalspule befindlicher Speicherchip (EEPROM) auf eine andere Adresse geschaltet wird. So würde die Lokalspule zwar vom System erkannt werden, aber es könnte sichergestellt werden, dass die Spule in diesem Zustand nicht mehr zu einer Messung verwendet werden kann. Alternativ kann eine Mechanik elektronische Bauteile der lokalspule, z.B. Induktivitäten, Kapazitäten oder Halbleiter, beim Entstecken durch Durchbrechen oder Abscheren zerstören.

Eine Zerstörung kann auch mit Hilfe von elektromagnetischen Feldern realisiert werden, anstatt über leitungsgebundene Spannungen/Ströme: Ein hoher Hochfrequenzpuls oder Gradientenpuls kann einen Puls hoher Leistung (Strom und/oder Spannung) in der Lokalspule induzieren, und so zum Defekt von Bauteilen (Sicherungen, Halbleiter, Dioden, Speicherbausteine, Induktivitäten oder Kapazitäten oder Widerstände) führen. Dieser hohe Puls wird bevorzugt kurz vor oder während der Patient am Ende der Messung aus dem Bore herausgefahren wird erzeugt.

Im Grunde können die Änderungen alle möglichen elektrischen Eigenschaften der Lokalspule (Widerstand etc.) umfassen, die über eine bleibende Änderung durch elektrische oder mechanische Zerstörung eines Bauteils in der Spule oder durch das Erzeugen oder Aufbrechen eines Kurzschlusses diese Eigenschaft der Lokalspule ändern und diese Eigenschaft maßgebend dafür ist, ob das System die Nutzung der geänderten Lokalspule zulässt.

Bevorzugt sind auch software-basierte Maßnahmen, insbesondere basierend auf einer individuellen Identifikationsnummer der Lokalspule. Bevorzugt ist dabei die Sperrung der Nutzung durch Identifikation der Spule. Dazu wird die Identifikationsnummer der Spule nach Erkennen der Lokalspule mit einer Datenbank abgeglichen. Falls die Lokalspule noch gültig ist, bleibt sie für eine bestimmte Zeitdauer oder bis zum Eintreten eines bestimmten Ereignisses (siehe oben "Ende der Messung") gültig und nutzbar. Danach wird sie aus der Liste der nutzbaren Spulen gestrichen.

Bevorzugt ist aber auch ein Abgleich der Nutzung der Seriennummern mit einer zentralen Datenbank. Das System fragt hierzu bei Nutzung der Spule bevorzugt erst bei einem Server nach, ob die Seriennummer der Lokalspule (noch) gültig ist. Danach bleibt die Lokalspule für eine bestimmte Zeit gültig und wird dann aus der zentralen Datenbank gelöscht. Sie ist damit an keinem System mehr nutzbar, welches mit dieser zentralen Datenbank informationstechnisch verknüpft ist. Bevorzugt ist auch die Verteilung der zentralen Datenbank lokal auf alle Scanner des MRT-Systems. Hier sollte jedoch ein regelmäßiger Abgleich erfolgen. Der Vorteil ist hier, dass der verwendete Scanner zum Zeitpunkt der Messung nicht unbedingt online sein muss.

Die Zuordnung von vorhandenen Spulen zu den Seriennummern kann bevorzugt über eine zusätzliche Identifikationsnummer ("CodeID") erfolgen, die der Lokalspule oder einem größeren Gebinde an Lokalspulen zugeordnet ist. Mit Hilfe dieser zusätzlichen Identifikationsnummer kann man sich als Käufer der Lokalspule oder des Spulengebindes authentifizieren und die Nutzung der Lokalspulen auf einem lokalen Scanner bzw. im Krankenhaus freischalten oder die Freischaltung kaufen. Dabei kann die zusätzliche Identifikationsnummer (z.B. eine Gebinde-Identifikationsnummer) zum Freischalten der zugehörigen Spulenidentifikationsnummern führen. Alternativ kann die zusätzliche Identifikationsnummer einen Schlüssel darstellen, der über einen festen Algorithmus im System die zugehörigen Spulenseriennummern erzeugt oder freischaltet. Aber auch hier sollte ein Abgleich mit einem zentralen Server erfolgen, um die Nutzung der Spulen auf anderen Scannern zu verhindern, indem die IDs gesperrt werden. Jede Spulen ID sollte stets nur einmal vorhanden sein und sollte nur einmal genutzt werden können.

Bevorzugt ist auch eine Änderung des Inhalts eines Speichers auf der Lokalspule. Nach dem Ende einer Messung oder auch bereits unmittelbar nach dem ersten Stecken der Lokalspule, wird der Inhalt des Speichers der Spule geändert, so dass die Spule nicht nochmal genutzt werden kann. Dazu kann insbesondere ein Zähler heruntergezählt werden, der die Nutzungszahl definiert. Das MRT-System prüft dann diesen Zähler. Es kann aber auch bevorzugt die Identifikationsnummer der Lokalspule gelöscht oder mit einer anderen Nummer überschrieben werden. Dies sollte so erfolgen, dass da mit einem Benutzer oder einem MRT-System angezeigt wird, dass die Spule bereits benutzt wurde. Vorteilhafter Weise sollten hierzu geschützte Speicherbausteine verwendet werden, da ansonsten Missbrauch durch einfaches Rückschreiben der Originaldaten möglich ist. Softwarebasierte Verfahren sind besonders vorteilhaft, wenn sie zusammen mit geschützten Schreib- bzw. Kommunikationsverfahren verwendet werden und erlauben theoretisch auch eine Begrenzung der Nutzungszahl auf mehr als eine einzige Nutzung, falls es dafür sinnvolle Anwendungsfälle gibt. Aus hygienischen Gründen kann eine Einzelnutzung bevorzugt sein.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen und Ausführungsbeispielen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Gemäß einem bevorzugten Verfahren wird die Lokalspule über ein Interface als Zwischenkomponente mit dem MRT-System verbunden. Ein solches Interface ist im Grunde im Stand der Technik bekannt. Gemäß der Erfindung wird bevorzugt ein besonderes Interface verwendet, welches dazu ausgelegt ist, das gezielte Ändern der mechanischen und/oder elektrischen Eigenschaft der Lokalspule vorzunehmen. Des Weiteren ist das Interface bevorzugt dazu ausgelegt, die Fertigstellung einer Messung zu ermitteln und die Änderungen der Lokalspule erst bei einer ermittelten Fertigstellung der Messung durchzuführen. Dieses Interface übernimmt also bevorzugt zusätzliche mechanische oder elektrische Funktionen zur gezielten Zerstörung der genutzten Lokalspule.

Gemäß einem bevorzugten Verfahren erfolgt ein gezieltes Ändern einer mechanischen und/oder elektrischen Eigenschaft der Lokalspule erst nach einer Feststellung der Messung. Dies hat den Vorteil, dass sichergestellt ist, dass auch wirklich mit der Lokalspule gemessen worden ist, bevor sie potentiell unbrauchbar gemacht wird.

Eine bevorzugte Indikation für eine Fertigstellung der Messung ist ein Ausstecken der Lokalspule vom System oder vom Interface. Dies sollte also zunächst ermittelt werden. Bei einer mechanischen Änderung kann die Kraft verwendet werden, die zum Ausstecken aufgebracht werden muss. Beispielsweise kann ein Anschluss der Lokalspule so beschaffen sein, dass er durch diesen Kraftaufwand abreißt oder es kann diese Kraft verwendet werden, um den Spulenleiter zu durchtrennen. Alternativ kann ein Ende der Messung ermittelt werden. Dies erfolgt bevorzugt durch Ermittlung, ob einen Kombination bestimmter typischer Abläufe am MRT-System stattgefunden hat, z.B. Patent in Bore eingefahren, Messung erfolgt, Patent wieder aus dem Bore ausgefahren, oder es werden Daten des MRT-Systems ausgewertet und Daten des MRT-Systems als Ende der Messung angesehen, die als explizites Messende festgelegt sind. Dies sollte definiert sein, bevor der Patient aus dem Bore herausgefahren wird. Es kann aber auch ein Ablauf einer vorgegebenen Gültigkeits-Zeitspanne zwischen Erkennen der Lokalspule oder dem Start der Messung ermittelt werden. Es wird bevorzugt mindestens einer der vorgenannten "Endpunkte" einer Messung bestimmt, um damit die Lebensdauer der Lokalspule zu begrenzen. Wird ein Ende der Messung festgestellt, kann eine elektrische Maßnahme, z.B. ein Leistungspuls, appliziert werden, um die Lokalspule unbrauchbar zu machen bzw. deren entsprechende Eigenschaft zu ändern.

Gemäß einem bevorzugten Verfahren umfasst das gezielte Ändern einer mechanischen und/oder elektrischen Eigenschaft der Lokalspule (insbesondere zum Hervorrufen eines Endes der Lebensdauer der Lokalspule) ein Unbrauchbarmachen der Lokalspule durch einen Nutzer, durch ein Interface oder durch das MRT-System. Alternativ oder zusätzlich umfasst das gezielte Ändern einer mechanischen und/oder elektrischen Eigenschaft der Lokalspule ein Ändern der Lokalspule durch ein Interface oder durch das MRT-System, insbesondere ihrer Erkennungsdaten, ihrer Impedanz, ihres Widerstandes und/oder ihrer Eigenfrequenz, dahingehend, dass sie vom MRT-System nicht mehr erkannt oder als nicht mehr gültig erkannt wird.

Gemäß einem bevorzugten Verfahren umfasst das gezielte Ändern einer mechanischen und/oder elektrischen Eigenschaft der Lokalspule eine Zerstörung einer elektrischen Komponente der Lokalspule, insbesondere eine Zerstörung des Hauptleiters, eines Steckkontaktes, einer Schmelzsicherung oder eines elektrischen Bauteils, insbesondere eines Widerstands, einer Diode, einer Induktivität oder einer Kapazität. Es ist dabei bevorzugt, dass durch Zerstörung einer Schmelzsicherung oder eines elektrischen Bauteils die Lokalspule im Hinblick auf ihre Eigenfrequenz verstimmt wird, wobei insbesondere durch diese Zerstörung ein Stromkreis aktiviert oder deaktiviert wird. Durch Öffnen einer Schmelzsicherung kann z.B. ein parallel geschaltetes Bauteil (Induktivität oder Kapazität) deaktiviert werden und dadurch die Lokalspule stark verstimmt werden. Ähnliches ist mit Dioden möglich, bei denen durch einen hohen Strom- oder Spannungspuls ein interner Kurzschluss erzeugt wird.

Gemäß einem bevorzugten Verfahren wird das gezielte Ändern einer mechanischen und/oder elektrischen Eigenschaft der Lokalspule durch eine mechanische Durchtrennung einer Komponente oder zumindest ihrer Zerstörung mittels einer Krafteinwirkung hervorgerufen. Alternativ oder zusätzlich wird das gezielte Ändern einer mechanischen und/oder elektrischen Eigenschaft der Lokalspule durch eine elektrische Zerstörung, insbesondere ein Durchbrennen, einer Komponente mittels eines leitungsgebundenen oder induzierten Stroms hervorgerufen. Dies kann durchaus ein Strom sein, der durch einen hohen Hochfrequenzpuls oder Gradientenpuls induziert worden ist. Dies kann bevorzugt mittels eines bevorzugten Interfaces erfolgen, welches zur Erzeugung eines solchen Leistungspulses ausgelegt ist. Es wird dabei bevorzugt ein Hauptleiter der Lokalspule oder eine Schmelzsicherung durchtrennt oder kurzgeschlossen oder eine Anzahl von Kontakten zur Lokalspule abgebrochen. Beispielsweise bildet das Interface einen mechanischen Eingriff in die Lokalspule, der beim Lösen (durch Ziehen oder Drücken eines Entriegelungsknopfes) des Interfaces dazu führt, dass der Hauptleiter durchtrennt wird. Der Hauptleiter ist dazu auf einer kurzen Strecke freitragend innerhalb der Lokalspule ausgeführt, so das der Leiter und seine dünne Tragestruktur leicht durchgebrochen werden kann.

Gemäß einem bevorzugten Verfahren umfasst das gezielte Ändern einer Eigenschaft der Lokalspule eine Änderung einer Spulenidentifikation, insbesondere einer Identifikationsnummer, wobei eine Datenschnittstelle der Lokalspule oder deren Kontakt zum MRT-System mechanisch oder elektrisch zerstört wird oder die Spulenidentifikation datentechnisch verändert wird. Die Spulenidentifikation kann z.B. eine Seriennummer sein oder eine individuelle Zahl, welche dieser Lokalspule zugeordnet worden ist. Es ist dabei bevorzugt, dass in dem Fall, in dem die Spulenidentifikation über eine elektrische Schnittstelle läuft, ein Leiter dieser Schnittstelle unterbrochen wird. Alternativ oder zusätzlich ist bevorzugt, dass die Spulenidentifikation durch mechanisches Unterbrechen oder Verbinden eines Kontaktes innerhalb der Lokalspule verändert wird, insbesondere in dem ein in der Lokalspule befindlicher Speicherchip auf eine andere Adresse geschaltet wird.

Gemäß einem bevorzugten Verfahren umfasst das gezielte Ändern einer elektrischen Eigenschaft der Lokalspule und/oder einer Information über die Lokalspule im MRT-System eine datentechnische Änderung einer Information. Dabei wird insbesondere in einem Speicherelement der Lokalspule oder des MRT-Systems eine individuelle Identifikationsnummer der Lokalspule geändert. Bevorzugt kann dort auch alternativ oder zusätzlich eine Information zur Gültigkeit einer Identifikationsnummer der Lokalspule geändert werden. Bevorzugt kann dort auch alternativ oder zusätzlich ein Schlüssel für eine datentechnische Kommunikation zwischen Lokalspule und MRT-System geändert werden. Bevorzugt kann dort auch alternativ oder zusätzlich eine Zählvariable zur Anzahl der noch möglichen Messungen mit dieser Lokalspule geändert werden.

Gemäß einem bevorzugten Verfahren wird die Identifikationsnummer der Lokalspule vor der Messung vom MRT-System mit einer Datenbank abgeglichen, in der eine Information über die Gültigkeit der Lokalspule enthalten ist. Falls die Lokalspule noch gültig ist, bleibt sie für eine bestimmte Zeitdauer oder bis zum Eintreten eines bestimmten Ereignisses (siehe oben "Ende der Messung") gültig und nutzbar. Danach wird sie aus der Liste der nutzbaren Lokalspulen gestrichen. Die Datenbank kann zentral für mehrere Scanner verwendet werden oder jeder Scanner kann eine eigene Datenbank besitzen, wobei diese Datenbanken bevorzugt regelmäßig miteinander abgeglichen werden, was den Vorteil hat, dass der betreffende Scanner zum Zeitpunkt der Messung nicht online sein muss.

Bevorzugt ist zusätzlich zur Identifikationsnummer eine Code-ID als weitere Identifikationsnummer auf Seiten der Lokalspule vorhanden. Diese Code-ID existiert dabei insbesondere zusätzlich zu Seriennummern einer Gruppe von Lokalspulen, insbesondere in Form einer Gebinde-Identifikationsnummer für eine Mehrzahl von Lokalspulen. Die Code-ID wird dabei bevorzugt vor der Messung vom MRT-System mit einer Datenbank abgeglichen, in der eine Information über die Gültigkeit der Code-ID enthalten ist. Die Zuordnung der vorhandenen Lokalspulen zu den Seriennummern kann über die Code-ID auf der Lokalspule (in der Lokalspule durch Abfrage über das System) oder einem größeren Gebinde an Wegwerfspulen erfolgen. Mit Hilfe dieser Nummer kann man sich als Käufer der Lokalspule oder des Lokalspulengebindes authentifizieren und die Nutzung der Lokalspulen auf dem lokalen Scanner bzw. im lokalen Krankenhaus freischalten oder die Freischaltung kaufen. Dabei kann die Gebinde-Identifikationsnummer zum Freischalten bzw. Herunterladen der zugehörigen Identifikationsnummern der Lokalspulen führen.

Bevorzugt stellt die Identifikationsnummer einen Schlüssel dar, der zur Datenkommunikation mit dem MRT-System dient. Dies kann z.B. über einen festen Algorithmus im System realisiert werden, der dann die zugehörigen Seriennummern der Lokalspulen erzeugt oder freischaltet. Aber auch hier sollte ein Abgleich mit einem zentralen Server gemacht werden, um die Nutzung der Lokalspulen auf anderen Scannern zu verhindern, indem die IDs gesperrt werden. Jede Lokalspulen ID ist nur einmal vorhanden und kann nur einmal genutzt werden.

Gemäß einem bevorzugten Verfahren findet eine Kommunikation zwischen der Lokalspule und dem MRT-System mittels eines Challenge-Response-Verfahrens statt. Bevorzugt umfassen Lokalspule und MRT-System dazu ein identisches Passwort und einen Hash-Generator, wobei bevorzugt die Lokalspule nach Empfang einer Zahl seitens des MRT-Systems diese Zahl mit dem Passwort verändert, einen Hash-Wert der geänderten Zahl erzeugt und den Hash-Wert an das MRT-System zurücksendet.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung eines Magnetresonanztomographiesystems gemäß einem Ausführungsbeispiel der Erfindung,
Figur 2 eine Lokalspule mit Schmelzsicherung,
Figur 3 eine Lokalspule mit Schmelzsicherung und Kondensator,
Figur 4 eine Lokalspule mit Diode (zum Kurzschließen),
Figur 5 den Leiter einer Lokalspule mit einem mechanischen Unterbrecher,
Figur 6 ein Kommunikationsverfahren zwischen einem Magnetresonanzscanner (oder Interface) und einem Server mit einer Datenbank,
Figur 7 ein Kommunikationsverfahren zwischen Scanner (oder Interface) und einer Datenbank (z.B. im Scanner), die optional durch einen Server auf den neuesten Stand gebracht werden kann,
Figur 8 ein Kommunikationsverfahren zwischen Scanner (oder Interface) und einer Datenbank mit zusätzlicher Code-ID (und einem optionalen Gebinde),
Figur 9 ein bevorzugtes Challenge-Response-Verfahren.

In den folgenden Figuren sind nur für die Erfindung wesentliche oder zu ihrem Verständnis hilfreiche Elemente eingezeichnet.

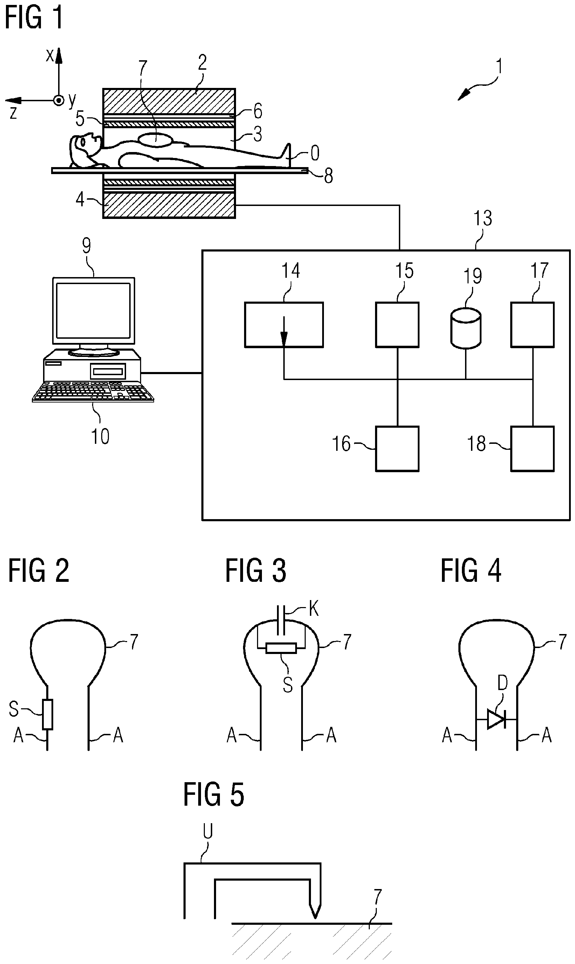

In Figur 1 ist grob schematisch ein Magnetresonanztomographiesystem 1 (MRT-System) dargestellt. Es umfasst zum einen den eigentlichen Magnetresonanzscanner 2 mit einem Untersuchungsraum 3 bzw. Patiententunnel, in den auf einer Liege 8 ein Patient oder Proband positioniert ist, in dessen Körper sich das eigentliche Untersuchungsobjekt O befindet. Auch wenn in dem dargestellten Beispiel das Untersuchungsobjekt O im Torso abgebildet ist, wird die Diffusions-Tensor-Bildgebung auch oft für Aufnahmen des Gehirns verwendet, da sie besonders gut zur Abbildung neurologischer Strukturen geeignet ist.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennensystem 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen 7 besteht (hier nur durch eine einzelne Lokalspule 7 am Körper symbolisiert). Das Grundfeldmagnetsystem 4 ist hier in üblicher Weise so ausgebildet, dass es ein Grundmagnetfeld in Längsrichtung des Patienten erzeugt, d. h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können.

Bei dem hier dargestellten MRT-System 1 handelt es sich um eine Ganzkörperanlage mit einem Patiententunnel, in den ein Patient komplett eingebracht werden kann. Grundsätzlich kann die Erfindung aber auch an anderen MRT-Systemen, z. B. mit seitlich offenem, C-förmigen Gehäuse, verwendet werden. Wesentlich ist nur, dass entsprechende Aufnahmen des Untersuchungsobjekts O angefertigt werden können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz oder einer Abfolge von mehreren Pulssequenzen zur Aufnahme mehrerer Schichten in einem interessierenden Volumenbereich des Untersuchungsobjekts innerhalb einer Messsitzung gesteuert. Eine solche Pulssequenz kann beispielsweise innerhalb eines Mess- oder Steuerprotokolls vorgegeben und parametrisiert sein. Üblicherweise sind verschiedene Steuerprotokolle für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden. Im vorliegenden Fall enthält die Steuereinrichtung 13 Pulssequenzen zur Akquisition der Rohdaten.

Zur Ausgabe der einzelnen HF-Pulse einer Pulssequenz weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend der vorgegebenen Pulssequenz die Gradientenpulse passend zu schalten, weist die Steuereinrichtung 13 eine Gradientensystemschnittstelle 16 auf. Die Sequenzsteuereinheit 14 kommuniziert in geeigneter Weise, z. B. durch Aussendung von Sequenzsteuerdaten SD, mit der Hochfrequenzsendeeinrichtung 15 und der Gradientensystemschnittstelle 16 zur Ausführung der Pulssequenz.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz PS vorgegebenen Auslesefenster koordiniert mittels des HF-Empfangsantennensystems 7 Magnetresonanz-Signale zu empfangen und so die Rohdaten zu akquirieren.

Eine Rekonstruktionseinheit 18 übernimmt hier die akquirierten Rohdaten und rekonstruiert daraus Magnetresonanz-Bilddaten. Auch diese Rekonstruktion erfolgt in der Regel auf Basis von Parametern, die in dem jeweiligen Mess- oder Steuerprotokoll vorgegeben sein können. Diese Bilddaten können dann beispielsweise in einem Speicher 19 hinterlegt werden.

Wie im Detail durch ein Einstrahlen von HF-Pulsen und das Schalten von Gradientenpulsen geeignete Rohdaten akquiriert und daraus MR-Bilder oder Parameter-Karten rekonstruiert werden können, ist dem Fachmann grundsätzlich bekannt und wird daher hier nicht näher erläutert.

Eine Bedienung der zentralen Steuereinrichtung 13 kann über ein Terminal 11 mit einer Eingabeeinheit 10 und einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanztomographie-Bilder angezeigt werden, und mittels der Eingabeeinheit 10, gegebenenfalls in Kombination mit der Anzeigeeinheit 9, können Messungen geplant und gestartet werden.

Das erfindungsgemäße Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten bzw. Parameterkarten, aber auch weitere Daten, wie beispielsweise patientenrelevante Daten oder Steuerprotokolle, austauschen zu können.

Wie durch ein Einstrahlen von HF-Pulsen und die Erzeugung von Gradientenfeldern geeignete Rohdaten akquiriert und daraus Magnetresonanztomographie-Bilder rekonstruiert werden können, ist dem Fachmann grundsätzlich bekannt und wird hier nicht näher erläutert.

Figur 2 zeigt eine Lokalspule 7 mit Schmelzsicherung S. Wird diese Schmelzsicherung S zerstört, z.B. durch einen Leistungspuls, sind die beiden Anschlusspins A nicht mehr miteinander verbunden und es kann kein Signal mehr durch die Lokalspule 7 aufgefangen werden.

Figur 3 zeigt eine Lokalspule 7 mit Schmelzsicherung S und Kondensator K. Wird diese Schmelzsicherung S zerstört, so überbrückt sie den Kondensator K nicht mehr und die Resonanzfrequenz der Lokalspule 7 ändert sich durch den Kondensator K.

Figur 4 zeigt eine Lokalspule 7 mit Diode D. Wird diese Diode D durch einen Leistungspuls kurzgeschlossen, so sind die beiden Anschlusspins A direkt über die Diode D miteinander verbunden und es kann kein Signal mehr durch die Lokalspule 7 aufgefangen werden.

Figur 5 zeigt den Leiter einer Lokalspule 7 mit einem mechanischen Unterbrecher U als Änderungseinheit U. Wird dieser Unterbrecher U auf den Leiter gedrückt, so wird dieser zerstört, was zu einer Zerstörung der Lokalspule 7 führt.

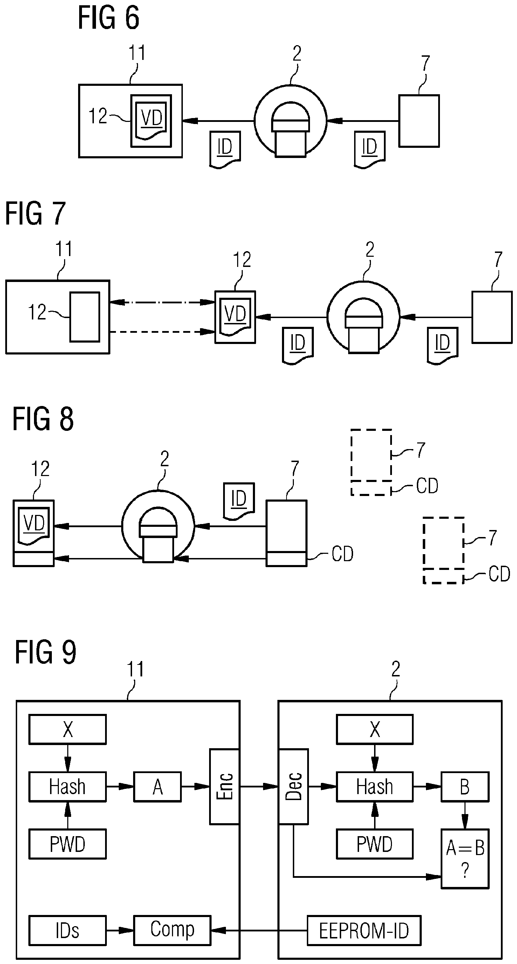

Figur 6 zeigt ein Kommunikationsverfahren zwischen einem Magnetresonanzscanner 2 (oder einem Interface) und einem Server 11 mit einer Datenbank 12. Die Lokalspule 7 sendet Identifikationsdaten ID, z.B. ihre Seriennummer, an den Magnetresonanzscanner 2, der diese wiederum an den Server 11 weiterleitet In der Datenbank 12 wird anhand von Vergleichsdaten VD überprüft, ob die Identifikationsdaten ID zu einer verwendbaren Lokalspule 7 gehören oder nicht. Ist die Lokalspule 7 verwendbar, ist eine Messung mit dem Magnetresonanzscanner 2 möglich und die entsprechenden Vergleichsdaten VD werden danach in der Datenbank 12 so geändert, dass eine weitere Messung mit derselben Lokalspule 7 nicht mehr möglich ist. Beispielsweise ist in der Datenbank 12 eine Liste mit Seriennummern (als Vergleichsdaten VD) gespeichert. Stimmen nun die von einer Lokalspule 7 gesendeten Identifikationsdaten ID mit einer dieser Seriennummern überein, ist eine Messung möglich, wenn nicht dann nicht. Nach einer Messung kann die betreffende Seriennummer einfach aus der Datenbank 12 gelöscht werden.

Figur 7 zeigt ein Kommunikationsverfahren zwischen einem Magnetresonanzscanner 2 (oder Interface) und einer Datenbank 12 (z.B. im Magnetresonanzscanner 2), die optional durch einen Server 11 auf den neuesten Stand gebracht werden kann. Im Grunde erfolgt eine Überprüfung der Lokalspule 7 wie in Figur 6 mit dem Unterschied, dass sich die Datenbank 12 direkt im Magnetresonanzscanner 2 befindet. Geänderte bzw. gelöschte Seriennummern können durch Austausch mit der Datenbank 12 im Server 11 anderen Magnetresonanzscannern 2 mitgeteilt werden. Die gestrichelten Pfeile deuten eine solche Kommunikation der Datenbanken 12 an. Die Datenbank 12 im Server 12 kann durch Informationen der Datenbanken 12 der Magnetresonanzscanner 2 auf den neusten Stand gebracht werden oder diese auf den neuesten Stand bringen.

Figur 8 zeigt ein Kommunikationsverfahren zwischen einem Magnetresonanzscanner 2 (oder Interface) und einer Datenbank 12 mit zusätzlicher Code-ID CD (und einem optionalen Gebinde). Im Grunde erfolgt eine Überprüfung der Lokalspule 7 wie in Figur 6 mit dem Unterschied, dass zusätzlich eine Code-ID CD gesendet wird, welche eine Freischaltung des Gebindes überprüfbar macht. Ist z.B. die Code-ID CD freigeschaltet, dann ist eine Messung mit den Lokalspulen 7 des Gebindes möglich, mit jeder jedoch nur ein Mal.

Figur 9 zeigt ein bevorzugtes Challenge-Response-Verfahren. Eine Zufallszahl ("X"), welche Server 11 und Magnetresonanzscanner 2 kennen, wird durch Einwirken eines Passworts ("PWD") verändert und der Hash-Wert des Ergebnisses berechnet. Der Hasch-Wert ("A") des Servers 11 wird an den Magnetresonanzscanner 2 gesendet und dort mit dem dort berechneten Hasch-Wert ("B") verglichen. Stimmen beide Hasch-Werte ("A" und "B") miteinander überein, so ist das Challenge-Response-Verfahren positiv und es können die Vergleiche (s. Figuren 6 bis 8) durchgeführt werden.

Es wird abschließend noch einmal darauf hingewiesen, dass es sich bei den vorhergehend detailliert beschriebenen Verfahren sowie bei der dargestellten Lokalspule 7 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können. Der Ausdruck "eine Anzahl" ist als "mindestens eins" zu verstehen.

## Patentansprüche

1. Verfahren zum Entwerten einer Lokalspule (7) für ein MRT-System (1) umfassend die Schritte:
- Durchführen einer Messung mit der Lokalspule (7) an dem MRT-System (1),
- gezieltes Ändern einer mechanischen und/oder elektrischen Eigenschaft der Lokalspule (7) und/oder einer Information über die Lokalspule (7) im MRT-System (1) dahingehend, dass eine Anzahl der mit dieser Lokalspule (7) möglichen Messungen reduziert wird und in dem Fall, dass nur die durchgeführte Messung mit der Lokalspule (7) möglich war, eine weitere Messung mit dieser Lokalspule (7) zumindest an dem MRT-System (1) nicht mehr möglich ist.

2. Verfahren nach Anspruch 1, wobei die Lokalspule (7) über ein Interface als Zwischenkomponente mit dem MRT-System (1) verbunden wird und das Interface das gezielte Ändern der mechanischen und/oder elektrischen Eigenschaft der Lokalspule (7) vornimmt und zuvor bevorzugt eine Fertigstellung der Messung ermittelt.

3. Verfahren nach einem der vorangehenden Ansprüche, wobei ein gezieltes Ändern einer mechanischen und/oder elektrischen Eigenschaft der Lokalspule (7) erst nach einer Feststellung einer Fertigstellung der Messung erfolgt, wobei bevorzugt als Indikation für eine Fertigstellung der Messung
- ein Ausstecken der Lokalspule (7) vom MRT-System (1) oder vom Interface ermittelt wird,
- ein Ende der Messung ermittelt wird, bevorzugt i) durch Ermittlung, ob eine Kombination bestimmter typischer Abläufe am MRT-System (1) stattgefunden hat, ii) durch Daten des MRT-Systems (1) als explizites Messende festgelegt ist, und/oder
- ein Ablauf einer vorgegebenen Gültigkeits-Zeitspanne zwischen Erkennen der Lokalspule (7) und dem Start der Messung ermittelt wird.

4. Verfahren nach einem der vorangehenden Ansprüche, wobei das gezielte Ändern einer mechanischen und/oder elektrischen Eigenschaft der Lokalspule (7)
- ein Unbrauchbarmachen der Lokalspule (7) durch einen Nutzer, ein Interface oder das MRT-System (1) umfasst, oder
- ein Ändern der Lokalspule (7) durch ein Interface oder das MRT-System (1), insbesondere ihrer Erkennungsdaten, ihrer Impedanz, ihres Widerstandes und/oder ihrer Eigenfrequenz, dahingehend umfasst, dass sie vom MRT-System (1) nicht mehr erkannt oder als nicht mehr gültig erkannt wird.

5. Verfahren nach einem der vorangehenden Ansprüche, wobei das gezielte Ändern einer mechanischen und/oder elektrischen Eigenschaft der Lokalspule (7) eine Zerstörung einer elektrischen Komponente der Lokalspule (7) umfasst, insbesondere eine Zerstörung eines Hauptleiters, eines Steckkontaktes (A), einer Schmelzsicherung (S) oder eines elektrischen Bauteils, insbesondere eines Widerstands, einer Diode (D), einer Induktivität oder einer Kapazität (K), bevorzugt wobei durch Zerstörung einer Schmelzsicherung (S) oder eines elektrischen Bauteils die Lokalspule (7) im Hinblick auf ihre Eigenfrequenz verstimmt wird, wobei insbesondere durch diese Zerstörung ein Stromkreis aktiviert oder deaktiviert wird.

6. Verfahren nach einem der vorangehenden Ansprüche, wobei das gezielte Ändern einer mechanischen und/oder elektrischen Eigenschaft der Lokalspule (7) durch eine mechanische Durchtrennung einer Komponente oder zumindest ihrer Zerstörung mittels einer Krafteinwirkung hervorgerufen wird und/oder durch eine elektrische Zerstörung, insbesondere ein Durchbrennen, einer Komponente mittels eines leitungsgebundenen oder induzierten Stroms hervorgerufen wird, insbesondere mittels eines bevorzugten Interfaces, bevorzugt wobei ein Hauptleiter der Lokalspule (7) oder eine Schmelzsicherung (S) durchtrennt oder kurzgeschlossen wird, oder eine Anzahl von Kontakten (A) zur Lokalspule (7) abgebrochen wird.

7. Verfahren nach einem der vorangehenden Ansprüche, wobei das gezielte Ändern einer Eigenschaft der Lokalspule (7) eine Änderung einer Spulenidentifikation umfasst, insbesondere einer Identifikationsnummer (ID), wobei eine Datenschnittstelle der Lokalspule (7) oder deren Kontakt zum MRT-System (1) mechanisch oder elektrisch zerstört wird oder die Spulenidentifikation datentechnisch verändert wird, bevorzugt wobei
- in dem Fall, in dem die Spulenidentifikation über eine elektrische Schnittstelle läuft, ein Leiter dieser Schnittstelle unterbrochen wird,
- die Spulenidentifikation durch mechanisches Unterbrechen oder Verbinden eines Kontaktes innerhalb der Lokalspule (7) verändert wird, insbesondere in dem ein in der Lokalspule (7) befindlicher Speicherchip auf eine andere Adresse geschaltet wird.

8. Verfahren nach einem der vorangehenden Ansprüche, wobei das gezielte Ändern einer elektrischen Eigenschaft der Lokalspule (7) und/oder einer Information über die Lokalspule (7) im MRT-System (1) eine datentechnische Änderung einer Information umfasst, insbesondere wobei in einem Speicherelement der Lokalspule (7) oder des MRT-Systems (1)
- eine individuelle Identifikationsnummer (ID) der Lokalspule (7) und/oder
- eine Information zur Gültigkeit einer Identifikationsnummer (ID) der Lokalspule (7) und/oder
- ein Schlüssel für eine datentechnische Kommunikation zwischen Lokalspule (7) und MRT-System (1) und/oder
- eine Zählvariable zur Anzahl der noch möglichen Messungen mit dieser Lokalspule (7),
geändert wird.

9. Verfahren nach Anspruch 8, wobei
- die Identifikationsnummer (ID) der Lokalspule (7) vor der Messung vom MRT-System (1) mit einer Datenbank (12) abgeglichen wird, in der eine Information über die Gültigkeit der Lokalspule (7) enthalten ist, und/oder
- zusätzlich zur Identifikationsnummer (ID) eine Code-ID (CD) als weitere Identifikationsnummer auf Seite der Lokalspule (7) vorhanden ist, und diese Code ID (CD) insbesondere zusätzlich zu Seriennummern einer Gruppe von Lokalspulen (7) existiert, insbesondere in Form einer Gebinde-Identifikationsnummer für eine Mehrzahl von Lokalspulen (7), und wobei die Code-ID (CD) vor der Messung vom MRT-System (1) mit einer Datenbank (12) abgeglichen wird, in der eine Information über die Gültigkeit der Code-ID enthalten ist, und/oder
- die Identifikationsnummer (ID) einen Schlüssel darstellt, der zur Datenkommunikation mit dem MRT-System (1) dient.

10. Verfahren nach einem der vorangehenden Ansprüche, wobei eine Kommunikation zwischen der Lokalspule (7) und dem MRT-System (1) mittels eines Challenge-Response-Verfahrens stattfindet, und bevorzugt Lokalspule (7) und MRT-System (1) ein identisches Passwort und einen Hash-Generator umfassen, wobei bevorzugt die Lokalspule (7) nach Empfang einer Zahl seitens des MRT-Systems (1) diese Zahl mit dem Passwort verändert, einen Hash-Wert der geänderten Zahl erzeugt und den Hash-Wert an das MRT-System (1) zurücksendet.

11. Lokalspule (7) für eine Messung mit einem MRT-System (1) umfassend eine Änderungseinheit (U) zum gezielten Ändern einer mechanischen und/oder elektrischen Eigenschaft der Lokalspule (7) dahingehend, dass eine Anzahl der möglichen Messungen reduziert wird und in dem Fall, dass nur die durchgeführte Messung mit der Lokalspule (7) möglich war, eine weitere Messung mit dieser Lokalspule (7) an dem MRT-System (1) nicht mehr möglich ist.

12. Lokalspule nach Anspruch 11, umfassend eine Speichereinheit mit einem geschützten Speicher, dessen Inhalt durch ein Element der Lokalspule (7) oder eines Interfaces geändert werden kann aber nicht durch einen Benutzer.

13. Lokalspule nach Anspruch 11 oder 12, welche eine RX-Spule oder eine TX-Spule oder eine RX/TX-Spule ist.

14. Lokalspule nach einem der Ansprüche 11 bis 13, umfassend eine Einheit zur Ermittlung einer Fertigstellung der Messung, mit der Lokalspule (7) an dem MRT-System (1), wobei die Lokalspule (7) dazu ausgelegt ist, dass nach Ermittlung einer Fertigstellung einer Messung automatisch das gezielte Ändern einer mechanischen und/oder elektrischen Eigenschaft der Lokalspule (7) bewirkt wird.

15. MRT-System (1) umfassend eine Änderungseinheit (U) zum gezielten Ändern einer mechanischen und/oder elektrischen Eigenschaft einer Lokalspule (7) dahingehend, dass eine Anzahl der möglichen Messungen reduziert wird und in dem Fall, dass nur die durchgeführte Messung mit der Lokalspule (7) möglich war, eine weitere Messung mit dieser Lokalspule (7) an dem MRT-System (1) nicht mehr möglich ist.
